Europäisches Patentamt

European Patent Office

Office européen des brevets

(19)

(11) Publication number: **0 304 332 B1**

(12) **EUROPEAN PATENT SPECIFICATION**

(45) Date of publication of patent specification: **14.07.93** (51) Int. Cl.5: **C11D 3/386**, C12N 9/98

(21) Application number: **88307725.7**

(22) Date of filing: **19.08.88**

(54) **Enzyme containing granulate and method for production thereof.**

(30) Priority: **21.08.87 DK 4356/87**

(43) Date of publication of application:
**22.02.89 Bulletin 89/08**

(45) Publication of the grant of the patent:
**14.07.93 Bulletin 93/28**

(84) Designated Contracting States:
**AT BE CH DE FR GB IT LI NL SE**

(56) References cited:
**FR-A- 2 357 301**
**GB-A- 2 167 758**

(73) Proprietor: **NOVO NORDISK A/S**
**Novo Allé**
**DK-2880 Bagsvaerd(DK)**

(72) Inventor: **Markussen, Erik Kjaer**
**Tornekrogen 18**
**DK-3500 Vaerloese(DK)**

(74) Representative: **Brown, John David et al**
**FORRESTER & BOEHMERT Franz-**
**Joseph-Strasse 38**
**W-8000 München 40 (DE)**

Rank Xerox (UK) Business Services
(3.10/3.6/3.3.1)

**Description**

The granulate according to the invention and a method for production thereof is a further development of the granulate and the method for production thereof described in US 4,106,991, which is carried out in a drum granulator. It is to be understood that the term drum granulator represents a broad category of granulator devices, comprising for instance pan granulators and granulation mixers.

Even if the known granulate possesses many advantageous characteristics the physical stability of the known granulate is open to improvement.

Surprisingly, according to the invention it has been found that a granulate built up upon a core, but otherwise substantially as indicated in US 4,106,991, exhibits improved physical stability and other improved characteristics, as will appear from the following.

Thus, the enzyme granulate according to the invention comprises a core surrounded by a coating comprising cellulose fibres or artificial fibres in an amount of 1.5 to 40% by weight, based on the dry weight of the granulate, except for the core, a binder in an amount of 0 - 15% by weight, based on the dry weight of the granulate, except for the core, a filler, and one or more granulating agents, whereby the core and/or the coating contains an enzyme. The cellulose fibres or the artificial fibres should possess sufficient flexibility to be adaptable to the shape of the cores.

Due to the introduction of the core, which can be very cheap, the amount of (expensive) cellulose fibres (or artificial fibres) and binder can be reduced below the level indicated in US 4,106,991. Thus, the overall cost of the granulate can be somewhat reduced in comparison to the US 4,106,991 prior art. Also, it has been found that the physical stability of the granulate is improved over the US 4,106,991 prior art, due to the reticular structure of the oriented fibres. Also, probably due to the interplay between the core and the fibres circumscribed around the core it has been found that it is possible to produce a granulate with a higher enzymatic activity in comparison to the prior art. Furthermore, as will be explained later in more detail, it is possible in a simple way to obtain the granulate in a very narrow particle size distribution, which can be tailor made to any need.

The core can be made up of any coherent, non crumbling material which will not cause any damage during the end use of the enzyme granulate. Thus, if the enzyme granulate is to be used as a detergent additive, the core could be inorganic or organic salts, which are easily or sparingly soluble in water, e.g. sodium chloride, sodium sulphate, sodium tripolyphosphate or sodium citrate. Also, in this case the core may be a particle of an already produced enzyme granulate. In this manner granulates with different enzymes in the core and the coating may be produced. If for instance the enzyme granulate is to be used as a nutrient additive, the core could be starch, sugar or protein. Thus, the granulate according to the invention can be designed in the following three manners.

| Core | Coating | |
|---|---|---|
| | enzyme | no enzyme |
| enzyme | X | X |
| no enzyme | X | |

The cellulose in fibrous form can be sawdust, pure fibrous cellulose, cotton, or other forms of pure or impure fibrous cellulose.

Several brands of cellulose in fibrous form are on the market, e.g. CEPO and ARBOCEL. In a publication from Svenska Tramjolsfabrikerna AB, "Cepo Cellulose Powder" it is stated that for Cepo S/20 cellulose the approximate maximum fibre length is 500 $\mu$, the approximate average fibre length is 160 $\mu$, the approximate maximum fibre width is 50 $\mu$ and the approximate average fibre width is 30 $\mu$. Also it is stated that CEPO SS/200 cellulose has an approximate maximum fibre length of 150 $\mu$, an approximate average fibre length of 50 $\mu$, an approximate maximum fibre width of 45 $\mu$ and an approximate average fibre width of 25 $\mu$. Cellulose fibres with these dimensions are very well suited for the purpose of the invention.

Typical artificial fibres are made of polyethylene, polypropylene, polyester, especially Nylon, polyvinylformal, and poly(meth)acrylic compounds.

The binders used in the process according to the invention are the binders conventionally used in the field of granulation with a high melting point or with no melting point at all and of a non waxy nature, e.g. polyvinyl pyrrolidone, dextrins, polyvinylalcohol, and cellulose derivatives, including for example hydroxypropyl cellulose, methyl cellulose or CMC. A granulate cannot be formed on the basis of cellulose, enzyme filler and a binder, as above defined, without the use of a granulating agent, as defined below.

All enzymes can be granulated by means of the process according to the present invention. Preferably, proteinases, amylases, lipases and cellulases are granulated according to the invention. Specific examples are ALCALASE® (a Bacillus licheniformis proteinase), ESPERASE® and SAVINASE® (microbial alkaline proteinases produced according to the British Pat. No. 1,243,784), CELLUZYME® (a Humicola insolens cellulase according to US patent No. 4,435,307), and TERMAMYL® (a Bacillus licheniformis amylase). The enzyme can be introduced into the granulator as a predried milled powder or as a solution, for example a concentrated enzyme solution prepared by ultrafiltration, reverse osmosis or evaporation.

The filler is used only for the purpose of adjusting to the intended enzyme activity in the finished granulate. Since the enzyme introduced into the granulator already contains diluent impurities which are considered as fillers additional filler is not always needed to standardize the enzymatic activity of the granulate. If a filler is used, it is usually organic or inorganic salts, which may be soluble or insoluble in water, e.g. $Na_2SO_4$, NaCl, $CaCO_3$, or minerals, silicates, e.g. kaolin or bentonite, but other indifferent fillers which do not interfere adversely with the later use of the product can be used.

The granulating agent is water and/or a waxy substance. The granulating agent is always used as a liquid phase in the granulation process; the waxy substance if present therefore is either dissolved or dispersed in the water or melted. By a waxy substance is understood a substance which possesses all of the following characteristics: (1) the melting point is between 30° and 100°C, preferably between 40° and 60°C, (2) the substance is of a tough and not brittle nature, and (3) the substance possesses substantial plasticity at room temperature.

Both water and waxy substance are granulating agents, i.e. they are both active during the formation of the granules, the waxy substance stays as a constituent in the finished granules, whereas the majority of the water is removed during the drying. Thus in order to refer all amounts to the finished, dry granules all percentages are calculated on the basis of total dry substances, which means that water, one of the granulating agents, is not added to the other constituents when calculating the percentage of water, whereas the waxy substance, the other granulating agent, has to be added to the other dry constituents when calculating the percentage of waxy substance. Examples of waxy substances are polyglycols, fatty alcohols, ethoxylated fatty alcohols, higher fatty acids, mono-, di- and triglycerolesters of higher fatty acids, e.g. glycerol monostearate, alkylarylethoxylates, and coconut monoethanolamide.

If a high amount of waxy substance is used, relatively little water should be added, and vice versa. Thus the granulating agent can be either water alone, waxy substance alone or a mixture of water and waxy substance. In case a mixture of water and waxy substance is used, the water and the waxy substance can be added in any sequence, e.g. first the water and then the waxy substance, or first the waxy substance and then the water or a solution or suspension of the waxy substance in the water. Also, in case a mixture of water and waxy substance is used, the waxy substance can be soluble or insoluble (but dispersable) in water.

If no water is used in the granulating agent, usually no drying is needed. In this case the granulating agent is a melted waxy material, and only cooling is needed to solidify the particles. In most cases, however, some drying is performed, and the drying is usually carried out as a fluid bed drying whereby small amounts of dust and small granules are blown away from the surface of the granules. However, any kind of drying can be used. In the instance where no water is used as a granulating agent, a flow conditioner or anticaking agent may be added to the granulate either before or after the cooling step, e.g. fumed silica, for instance the commercial products AEROSIL or CAB-O-SIL.

The granulator can be any of the known types of mixing granulators, drum granulators, pan granulators or modifications of these. If a mixing granulator is used, for example a mixing drum from the German Company Gebr. Lödige Maschinen G.m.b.H, 479 Paderborn, Elsenerstrasse 7-9, DT, it is preferred that small rotating knives are mounted in the granulator in order to compact the granules.

Furthermore, facultative additives as coloring agents, pigments, disintegrants, or enzyme stabilizers, e.g. antioxidants, may be added.

In a preferred embodiment of the granulate according to the invention the core is of a shape corresponding to a maximum ratio between the largest and the smallest dimension of around 3, preferably around 2, more preferably around 1.5. In this embodiment a satisfactory coating around the core can always be obtained.

In a preferred embodiment of the granulate according to the invention the amount of the core is between 5 and 75% by weight, preferably between 10 and 50% by weight of the granulate, most preferably between 15 and 40% by weight of the granulate. If the amount of the core is above 75%, the physical strength tends to be unsatisfactory, and if the amount of the core is less than 5%, the advantages associated with the core are not obtained.

In a preferred embodiment of the granulate according to the invention the core has a mean particle size between 100 and 1000 μm, preferably 200-700 μm. These are the particle sizes most often required by the users.

In a preferred embodiment of the granulate according to the invention the cellulose fibres or the artificial fibres have an average fibre length of 50-2000 μm, preferably 100-1000 μm, and an average fibre width of 5-50 μm, preferably 10-40 μm. Hereby a satisfactory mechanical strength is obtained.

In a preferred embodiment of the granulate according to the invention the amount of cellulose fibres or artificial fibres are between 4 and 20% by weight based on the dry weight of the granulate, except for the core. Below 4% by weight of fibres no satisfactory fibre effect is obtained, and above 20% by weight of fibres the production gets cumbersome and the product expensive.

In a preferred embodiment of the granulate according to the invention the core and the coating contains an enzyme, and the core enzyme is another enzyme than the coating enzyme. This embodiment represents a broadening of the possibilities for sequential treatment in a washing solution.

In a preferred embodiment of the granulate according to the invention the enzyme is a protease, an amylase, a lipase, a cellulase, or an oxidase. These are the most common enzymes in granulate form for industrial purposes.

In a preferred embodiment of the granulate according to the invention the filler consists of or comprises inorganic salts. Hereby a cheap granulate is obtained.

Also, the invention comprises a method for production of the granulate according to the invention.

The method for the production of the enzyme containing granulate according to the invention comprises the introduction into a granulator of a core, 1.5 to 40% by weight of cellulose fibres or artificial fibres, from 0 to 15% by weight of a binder, enzyme and filler in an amount which generates the intended enzyme activity in the finished granulate, a fluid granulating agent consisting of a waxy substance and/or water, in an amount in the range of from 5 to 70% by weight, whereby the maximum amount of waxy substance is 70% by weight and the maximum amount of water is 70% by weight, all percentages referring to the total amount of dry substances, except for the core, the sequence of the introduction of the different material being arbitrary, except that at least the major part of the granulating agent is introduced after at least a substantial part of the dry substances is introduced into the granulator, whereby the granulation is preferably performed at a temperature between 20 and 70°C, whereafter the granulate if necessary is dried.

In case a granulate, which contains enzyme in the core only, is to be produced, it is to be understood that no enzyme should be added except for the enzyme already present in the core.

The introduction of a core and auxiliary agents which are either dissolved or suspended as extremely fine particles, in a particulate material produced with a fluidized bed is known, but the introduction of a core and cellulose fibres or artificial fibres in relation to a granulate produced by drum granulation is novel. It is assumed that the new combination of the core and the cellulose fibres or artificial fibres, which can circumscribe themselves around the core as a coherent reticular structure, is the reason for the extraordinary high physical strength of the granulate according to the invention.

The method according to the invention can be performed both batchwise and continuously.

The invention will be illustrated by the following examples.

The activity units used in the examples are defined as follows.

| enzyme | activity unit | Definition indicated in |
|---|---|---|
| proteolytic | KNPU | AF 220/1-GB |
| amylolytic | KNU | AF 215/1 GB |
| lipolytic | LU | AF 95/4-GB |

On request these AF publications can be obtained from NOVO INDUSTRI A/S, Novo Allé, 2880 Bagsvaerd, Denmark.

In the examples the core material exhibited a cubical shape in case the core was NaCl, and a rounded, irregular shape in case the core was $Na_2SO_4$.

**EXAMPLE 1**

9.0 kg of NaCl core material with a particle size between 250 and 300 μm is introduced into a Lödige mixer FM 130 D/1MZ and is mixed with

5.2 kg of SAVINASE concentrate (34 KNPU/g)
3.0 kg of fibrous cellulose, Arbocel BC 200
1.2 kg of $TiO_2$
1.5 kg of adhesive carbohydrate
10.1 kg of finely ground $Na_2SO_4$

The total charge of 30 kg of dry matter is sprayed with 4.0 kg of water as described in Example 1 in US patent No. 4,106,991, and a post granulation for 5 minutes is performed with the rapidly rotating set of knives (vide fig. 3 in Danish patent No. 146857, corresponding to US patent No. 4,106,991), whereby the powder components are distributing themselves evenly and in a compact manner around the core surfaces. The moist granulate is dried in a fluid bed, whereby a product is obtained with the following properties.

Core: 30%, shell: 70%

Enzyme activity: 5,3 KNPU/g

Particle size distribution:

3.5% > 1000 $\mu$m
5.0% > 850 -
6.8% > 710 -
11.0% > 600 -
17.0% > 500 -
42.0% > 420 -
65.0% > 355 -
77.0% > 300 -
90.0% > 250 -
3.5% < 210 -
1.9% < 180 -
0.1% < 150 -

Mean diameter:

390 $\mu$m

The amount of dust and the physical strength are measured according to two different methods:

A. Elutriation dust method, as described in NOVO publication AF 129/3-GB, available on request from NOVO INDUSTRI A/S.

B. Novo attrition dust method, as described in NOVO publication AF 225/1-GB, available on request from NOVO INDUSTRI A/S.

It appears from the following table that the values in regard to dust and physical strength of the granulate according to this example is superior to the corresponding prior art values.

| Method | Invention, this example | | Prior art (US 4,106,991) | |
|--------|-------------|----------------------|------------|----------------------|
|        | total, mg   | active, $\mu$g (4 KNPU/g) | total, mg | active, $\mu$g (4 KNPU/g) |
| A      | 15.4        | 8800                 | 10 - 30    | 10,000-20,000        |
| B      | 11.9        | 4100                 | 10 - 20    | 10,000-20,000        |

## EXAMPLE 2

7.5 kg of $Na_2SO_4$ core material with a particle size between 300 and 500 $\mu$m is introduced into a Lödige mixer FM 130 D/1MZ and mixed with
9.0 kg of SAVINASE concentrate (28 KNPU/g)
3.38 kg of fibrous cellulose Arbocel BC 200
0.9 kg of $TiO_2$
7.5 kg of finely ground $Na_2SO_4$

The total charge is sprayed with 6.4 kg of binder solution (a 27% aqueous solution of adhesive carbohydrates). The granulate is processed and dried as described in Example 1, whereby a product is obtained with the following properties:

Core: 25%, shell 75%

Enzyme activity: 8.0 KNPU/g

Particle size distribution:

6.7% > 1000 $\mu$m

7.5% > 850 -
11.0% > 710 -
16.0% > 600 -
34.0% > 500 -
62.0% > 425 -
78.0% > 355 -
90.0% > 300 -
4.0% < 250 -
1.9% < 210 -
0.9% < 180 -
Mean diameter:
450 $\mu$m

The amount of dust and the physical strength appears from the following table.

| Method | total, mg | active, $\mu$g (4 KNPU/g) |
|--------|-----------|---------------------------|
| A | 3.8 | 1390 |
| B | 9.4 | 3900 |

**EXAMPLE 3**

6.0 kg of trisodium citrate . $2H_2O$ core material with particles of rounded, irregular shape and a particle size distribution as indicated below is introduced into a Lödige mixer FM 50 and is mixed with
2.2 kg of SAVINASE concentrate (38 KNPU/g)
0.6 kg of $TiO_2$
0.7 kg of adhesive carbohydrate
1.5 kg of fibrous cellulose Arbocel BC 200
5.0 kg of finely ground $Na_2SO_4$
The total charge is sprayed with 2.0 kg of water, and processing and drying is carried out as indicated in Example 1, whereby a product is obtained with the following properties:

```
Particle size distribution:  Sodium citrate        product
                             core
                                               15   > 1000  μm
                                               19   >  850  -
                                               26   >  707  -
                             1.2               42   >  600  -
                             20                64   >  500  -
                             50                83   >  420  -
                             70                92   >  355  -
                             20               1.7   <  300  -
                             10               0.5   <  250  -

Mean diameter:                                      565  μm
```

The amount of dust and the physical strength appears from the following table.

| Method | total, mg | active, $\mu$g (4 KNPU/g) |
|--------|-----------|---------------------------|
| A | 4.8 | 4800 |

**EXAMPLE 4**

In this example the shell is placed onto the core in two steps, as indicated in the following.

Step 1

7.5 kg of sodium sulphate core material with particle sizes between 300 and 500 $\mu$m is introduced into a Lödige mixer FM 50 and is mixed with

4.5 kg of SAVINASE concentrate (28 KNPU/g)

1.7 kg of fibrous cellulose, Arbocel BC 200

0.45 kg of $TiO_2$

3.75 kg of finely ground $Na_2SO_4$

The mixed dry components are sprayed with 3.0 kg of a binder solution (a 27% aqueous solution of adhesive carbohydrates), and the granulate is otherwise treated as in Example 1, but without the drying step.

Step 2

Without removing the moist granulate from step 1 from the mixer it is mixed with

4.5 kg of SAVINASE concentrate (28 KNPU/g)

1.7 kg of fibrous cellulose, Arbocel BC 200

0.45. kg of $TiO_2$

3.75 kg of finely ground $Na_2SO_4$

whereafter 3.0 kg of the same binder solution as the one used in step 1 is sprayed onto the contents of the mixer. The granulate is processed and dried as in Example 1, whereby a product is obtained with the following properties:

Core: 25%, shell 75%

Enzyme activity: 8.7 KNPU/g

Particle size distribution:

4.8% > 1000 $\mu$m

6.3% > 850 -

9.5% > 707 -

20.0% > 600 -

47.0% > 500 -

71.0% > 425 -

82.0% > 355 -

89.0% > 300 -

5.5% < 250 -

3.0% < 210 -

1.4% < 180 -

0.6% < 150 -

Mean diameter:

490 $\mu$m

The amount of dust and the physical strength appears from the following table.

| Method | total, mg | active, $\mu$g (4 KNPU/g) |
|--------|-----------|---------------------------|
| A | 2.0 | 1300 |
| B | 8.4 | 7100 |

**EXAMPLE 5**

15 kg of a protease core (produced according to US patent no. 4,106,991, Example 1, except that $Na_2SO_4$ was used as a dilution agent rather than NaCl, and that SAVINASE was used as the proteolytic enzyme rather than ALCALASE) with a particle core of 300-900 $\mu$m and with a nearly spherical shape was introduced into a Lödige mixer of type FM 50. At this stage the proteolytic core is provided with a coating containing an amylolytic enzyme (BAN) produced by means of B. amyloliquefaciens by mixing the core with a powder.

The composition of the powder is:

0.90 kg of BAN concentrate (925 KNU/g)

0.50 kg of fibrous cellulose, DIACEL 200

0.25 kg of $TiO_2$

2.90 kg of $Na_2SO_4$

The charge consisting of cores and powder is mixed, and 2.05 kg of a 27.3% of an aqueous solution of adhesive carbohydrate is sprayed thereon.

The granulate is treated and dried as described in Example 1, whereby a granulate with the following characteristics is obtained:

Enzyme activity:

4.7 KNPU/g (proteolytic)

36.0 KNU/g (amylolytic)

Particle distribution:

0.8% > 1000 $\mu$m

13.5% > 850 -

41.1% > 707 -

66.4% > 600 -

86.7% > 500 -

95.7% > 425 -

98.7% > 355 -

0.06% < 300 -

Mean diameter:

660 -

Dust content: (in 300 - 900 $\mu$m fraction)

| Method | Granulate according to example | | | core | | |
|--------|------------|------------|------------|---------|---------|---------|
| A: | 13.0 mg 13.5 - | 1320 $\mu$g 452 - | 4.0 KNPU/g 240 KPU/g | 25.1 mg | 8040 $\mu$g | 4.0 KNPU/g |
| B: | 1.6 mg 1.7 - | 361 $\mu$g 327 - | 4.0 KNPU/g 240 KNU/g | 14.0 mg | 5500 $\mu$g | 4.0 KNPU/g |

**EXAMPLE 6**

10 kg of SAVINASE cores of the same kind as the SAVINASE cores described in Example 5 is coated with a powder containing an amylolytic enzyme, whereby the powder forms coatings around the cores. The composition of the powder is as follows:

1.2 kg of BAN (925 KNU/g)

0.5 kg of fibrous cellulose, DIACEL 200

0.2 kg of titanium dioxide

2.3 kg of $Na_2SO_4$

The charge consisting of cores and powder is mixed and heated to 40°C, whereafter a solution of 1.2 kg of PEG in 0.6 kg of water is sprayed onto the charge. The granulate is treated and dried as described in Example 1. After drying the granulate is cooled to room temperature, whereby the wax solidifies. Hereby a granulate is obtained with the following characteristics:

Activity:

5.4 KNPU/g

72.0 KNU/g

Particle distribution:

3.3% >1000 $\mu$m

17.3% > 850 -

40.1% > 707 -

58.9% > 600 -

78.2% > 500 -

91.7% > 425 -
97.7% > 355 -
0.9% < 300 -
Mean diameter:
630 -
Dust content: (in 300 - 900 $\mu$m fraction)

| Method | Granulate according to example | | | core | | |
|---|---|---|---|---|---|---|
| A: | 24.8 mg<br>1.1 - | 217 $\mu$g<br>4600 - | 4.0 KNPU/g<br>240 KNU/g | 21.3 mg | 10800 $\mu$g | 4.0 KNPU/g |
| B: | 29.5 mg<br>30.3 - | 1160 $\mu$g<br>12800 - | 4.0 KNPU/g<br>240 KNU/g | 618.0 mg | 770000 $\mu$g | 4.0 KNPU/g |

**EXAMPLE 7**

10 kg of a $Na_2SO_4$ core with particle size 300-500 $\mu$m is introduced into a Lödige mixer, type FM 50, in which the core is provided with a coating containing an amylolytic enzyme by means of a three step process, in the following manner.

Initially 10 kg of cores are mixed with a powder consisting of
0.6 kg of BAN (750 KNU/g)
0.3 kg of fibrous cellulose, DIACEL 200
0.2 kg of titanium dioxide
1.3 kg of $Na_2SO_4$
For the sake of brevity this powder is referred to in the following as 2.4 kg of powder $P_1$.

The mixture of cores and powder is heated to 65°C, and this temperature is maintained throughout the entire process.

Subsequently 0.7 kg of pure melted PEG 4000 with a temperature of 70°C is sprayed onto the heated mixture of cores and powder. A granulating treatment is carried out as indicated in Example 1, but just for 1 minute.

Now another 2.4 kg of powder $P_1$ is introduced into the mixer, followed by 0.5 kg of PEG 4000, and a 1 minute granulating treatment as indicated above.

Then another 2.4 kg of powder $P_1$ is introduced into the mixer, followed by 1.2 kg of PEG 4000, and a 1 minute granulating treatment as indicated above.
Activity:
63 KNU/g
Particle size distribution:
0.3% >1000 $\mu$m
0.5% > 850 -
4.3% > 707 -
24.4% > 600 -
54.5% > 500 -
73.2% > 425 -
87.1% > 355 -
97.4% < 300 -
0.9% < 250 -
Mean diameter:
550 $\mu$m
Dust content: (in 300 - 900 $\mu$m fraction)
Method:
A: 2.0 mg 327 $\mu$g à 240 KNU/g
B: 4.6 - 717 - - 240 -

**EXAMPLE 8**

13 kg of a $Na_2SO_4$ based core of lipase produced according to Danish patent application no. 4499/87 with an activity of 26000 LU/g is introduced into a Lödige mixer type FM 50, in which the core is provided with a coating containing SAVINASE protease in a three step process, as follows:

The 13 kg of lipase cores are mixed with a powder consisting of

1.00 kg of ground SAVINASE concentrate of 39.5 KNPU/g

0.12 kg of fibrous cellulose, DIACEL 200

0.09 kg of titanium dioxide

0.91 kg of $Na_2SO_4$

For the sake of brevity this powder is referred to in the following as 2.12 kg of powder $P_2$.

The processing temperature is 20-30°C.

Then 0.9 kg of a binder solution which is a 25% aqueous solution of an adhesive carbohydrate is sprayed onto the mixture in the Lödige mixer.

Subsequently another 2.12 kg of powder $P_2$ is introduced into the mixer, followed by 0.7 kg of the above indicated binder solution, which is introduced by spraying.

Then another 2.12 kg of powder $P_2$ is introduced into the mixer, followed by 1.15 kg of binder solution, which is introduced by spraying. Finally granulation, compaction and drying is carried out as indicated in Example 1.

A granulate with the following properties is obtained:

Core: 67% by weight     coating: 33% by weight

Activity:

17.900 LU/g (lipolytic)

6.0 KNPU/g (proteolytic)

Particle size distribution:

2.3% > 1000 $\mu$m

12.0% > 850 -

29.1% > 707 -

48.7% > 600 -

71.5% > 500 -

86.5% > 425 -

95.1% > 355 -

3.0% < 300 -

Mean diameter:

590 $\mu$m

Dust content: (in 300 - 900 $\mu$m fraction)

| Method | Granulate according to example | core |
|---|---|---|
| A: | 3.9 mg   3360 $\mu$g     4.0 KNPU/g | |
| | 6.0 -         0 $\mu$g LU/filter | 32.4 mg   200 LU/filter |
| B: | 1.0 mg   1340 $\mu$g     4.0 KNPU/g | |
| | 1.7 -         0 $\mu$g LU/filter | 29.0 mg   142 LU/filter |

The features disclosed in the foregoing description, in the following claims and/or in the accompanying drawings may, both separately and in any combination thereof, be material for realising the invention in diverse forms thereof.

10

**Claims**

1. Enzyme containing granulate comprising a core surrounded by a coating comprising cellulose fibres or artificial fibres in an amount of 1.5 to 40% by weight, based on the dry weight of the granulate except for the core, a binder in an amount of 0 - 15% by weight, based on the dry weight of the granulate, except for the core, a filler, and one or more granulating agents, whereby the core and/or the coating contains an enzyme.

2. Granulate according to Claim 1, wherein the core is of a shape corresponding to a maximum ratio between the largest and the smallest dimension of around 3, preferably around 2, more preferably around 1.5.

3. Granulate according to Claims 1 - 2, wherein the amount of the core is between 5 and 75% by weight, preferably between 10 and 50% by weight of the granulate, most preferably between 15 and 40% by weight of the granulate.

4. Granulate according to Claims 1 - 3, wherein the core has a mean particle size between 100 and 1000 $\mu$m, preferably 200-700 $\mu$m.

5. Granulate according to Claims 1 - 4, wherein the cellulose fibres or the artificial fibres have an average fibre length of 50-2000 $\mu$m, preferably 100-1000 $\mu$m, and an average fibre width of 5-50 $\mu$m, preferably 10-40 $\mu$m.

6. Granulate according to Claims 1 - 5, wherein the amount of cellulose fibres or artificial fibres are between 4 and 20% by weight based on the dry weight of the granulate, except for the core.

7. Granulate according to Claims 1 - 6, wherein the core and the coating contains an enzyme, and the core enzyme is another enzyme than the coating enzyme.

8. Granulate according to Claims 1 - 7, wherein the enzyme is or the enzymes are a protease, an amylase, a lipase, a cellulase, and/or an oxidase.

9. Granulate according to Claims 1 - 8, wherein the filler consists of or comprises inorganic salts.

10. Method for production of the enzyme containing granulate according to Claims 1 - 9, which process comprises the introduction into a granulator of a core, 1.5 to 40% by weight of cellulose fibres or artificial fibres, from 0 to 15% by weight of a binder, enzyme and filler in an amount which generates the intended enzyme activity in the finished granulate, a fluid granulating agent consisting of a waxy substance and/or water, in an amount in the range of from 5 to 70% by weight, whereby the maximum amount of waxy substance is 70% by weight and the maximum amount of water is 70% by weight, all percentages referring to the total amount of dry substances, except for the core, the sequence of the introduction of the different material being arbitrary, except that at least the major part of the granulating agent is introduced after at least a substantial part of the dry substances is introduced into the granulator, whereby the granulation is preferably performed at a temperature between 20 and 70°C, whereafter the granulate if necessary is dried.

**Patentansprüche**

1. Enzymhaltiges Granulat, das einen Kern umfaßt, der von einer Umhüllung umgeben ist, die Cellulosefasern oder Kunstfasern in einer Menge von 1,5 bis 40 Gew.-%, bezogen auf das Trockengewicht des Granulats, ausgenommen den Kern, ein Bindemittel in einer Menge von 0 - 15 Gew.-%, bezogen auf das Trockengewicht des Granulats, mit Ausnahme des Kerns, einen Füllstoff und ein oder mehrere Granuliermittel umfaßt, wobei der Kern und/oder die Umhüllung ein Enzym enthält.

2. Granulat nach Anspruch 1, wobei der Kern von einer Form ist, die einem maximalen Verhältnis zwischen der größten und der kleinsten Abmessung von etwa 3, vorzugsweise etwa 2, bevorzugter etwa 1,5 entspricht.

3. Granulat nach Ansprüche 1 - 2, wobei die Menge des Kerns zwischen 5 und 75 Gew.-%, vorzugsweise zwischen 10 und 50 Gew.-% des Granulats, am bevorzugtesten zwischen 15 und 40 Gew.-% des Granulats liegt.

4. Granulat nach Ansprüchen 1 - 3, wobei der Kern eine mittlere Teilchengröße zwischen 100 und 1.000 $\mu$m, vorzugsweise 200 - 700 $\mu$m besitzt.

5. Granulat nach Ansprüchen 1 - 4, wobei die Cellulosefasern oder die Kunstfasern eine mittlere Faserlänge von 50 - 2.000 $\mu$m, vorzugsweise 100 - 1.000 $\mu$m, und mittlere Faserbreite von 5 - 50 $\mu$m vorzugsweise 10 - 40 $\mu$m besitzen.

6. Granulat nach Ansprüchen 1 - 5, wobei die Menge an Cellulosefasern und Kunstfasern zwischen 4 und 20 Gew.-% liegt, bezogen auf das Trockengewicht des Granulats, ausgenommen den Kern.

7. Granulat nach Ansprüchen 1 - 6, wobei der Kern und die Umhüllung ein Enzym enthält und daß das Kernenzym ein anderes Enzym als das Umhüllungsenzym ist.

8. Granulat nach Ansprüchen 1 - 7, wobei das Enzym oder die Enzyme eine Protease, eine Amylase, eine Lipase, eine Cellulase und/oder eine Oxidase ist (sind).

9. Granulat nach Ansprüchen 1 - 8, wobei der Füllstoff aus anorganischen Salzen besteht oder diese umfaßt.

10. Verfahren zur Herstellung des enzymhaltigen Granulats nach Ansprüchen 1 - 9, wobei das Verfahren umfaßt, daß in einen Granulator ein Kern, 1,5 bis 40 Gew.-% Cellulosefasern oder Kunstfasern, von 0 bis 15 Gew.-% eines Bindemittels, Enzym und Füllstoff in einer Menge, welche die beabsichtigte Enzymaktivität im fertiggestellten Granulat erzeugt, ein fluides Granuliermittel, das aus einer wachsartigen Substanz und/oder Wasser besteht, in einer Menge im Bereich von 5 bis 70 Gew.-%, wobei die maximale Menge an wachsartiger Substanz 70 Gew.-% ist und die maximale Menge an Wasser 70 Gew.-% ist, eingebracht werden, wobei alle Prozentangaben sich auf die Gesamtmenge an Trockensubstanzen bezieht, ausgenommen den Kern, wobei die Reihenfolge des Einbringens des unterschiedlichen Materials willkürlich ist, mit der Ausnahme, daß wenigstens der Hauptteil des Granuliermittels eingebracht wird, nachdem wenigstens ein beträchtlicher Teil der Trockensubstanzen in den Granulator eingebracht ist, wobei die Granulierung vorzugsweise bei einer Temperatur zwischen 20 und 70°C durchgeführt wird, wonach das Granulat, falls erforderlich, getrocknet wird.

**Revendications**

1. Granules contenant une enzyme, comprenant un noyau entouré par un enrobage comprenant des fibres de cellulose ou des fibres artificielles en une proportion de 1,5 à 40 % en poids relativement au poids sec d'un granule à l'exception du noyau, un liant en une proportion de 0 à 15 % en poids relativement au poids sec d'un granule à l'exception du noyau, une charge et un ou plusieurs agents de granulation, le noyau et/ou l'enrobage contenant une enzyme.

2. Granules selon la revendication 1, dans lesquels le noyau a une forme correspondant à un rapport maximal entre la plus grande et la plus petite dimensions d'environ 3, de préférence d'environ 2 et mieux d'environ 1,5.

3. Granules selon les revendications 1 ou 2, dans lesquels la proportion du noyau est entre 5 et 75 % en poids du granule, de préférence entre 10 et 50 % du poids, mieux entre 15 et 40 % du poids du granule.

4. Granules selon les revendications 1 à 3, dans lesquels le noyau a une granulométrie moyenne entre 100 et 1 000 $\mu$m, de préférence entre 200 et 700 $\mu$m.

5. Granules selon les revendications 1 à 4, dans lesquels les fibres de cellulose ou les fibres artificielles ont une longueur moyenne des libres de 50 à 2 000 $\mu$m, de préférence de 100 à 1 000 $\mu$m, et une largeur moyenne des fibres de 5 à 50 $\mu$m, de préférence de 10 à 40 $\mu$m.

**6.** Granules selon les revendications 1 à 5, dans lesquels la proportion des libres de cellulose ou des fibres artificielles est entre 4 et 20 % en poids, relativement au poids sec des granules à l'exception des noyaux.

**7.** Granules selon les revendications 1 à 6, dans lesquels le noyau et l'enrobage contiennent une enzyme et l'enzyme du noyau est une enzyme autre que l'enzyme de l'enrobage.

**8.** Granules selon les revendications 1 à 7, dans lesquels l'enzyme est ou les enzymes sont une protéase, une amylase, une lipase, une cellulase et/ou une oxydase.

**9.** Granules selon les revendications 1 à 8, dans lesquels la charge est constituée de sels minéraux ou comprend des sels minéraux.

**10.** Procédé pour préparer les granules contenant une enzyme selon les revendications 1 à 9, lequel procédé comprend l'introduction, dans un granulateur, de noyaux, de 1,5 à 40 % en poids de fibres de cellulose ou de fibres artificielles, de 0 à 15 % en poids d'un liant, d'une enzyme et d'une charge en une quantité qui établit l'activité enzymatique désirée dans les granules finis, d'un agent de granulation fluide constitué d'une substance cireuse et/ou d'eau, en une proportion dans la gamme de 5 à 70 % en poids, la proportion maximale de substance cireuse étant de 70 % en poids proportion maximale d'eau étant de 70 % en poids, tous les pourcentages étant relatifs à la quantité totale des substances sèches à l'exception des noyaux, l'ordre d'introduction des différentes matières étant quelconque, si ce n'est qu'au moins la majeure partie de l'agent de granulation est introduite après qu'au moins une partie importante des substances sèches soit introduite dans le granulateur, la granulation étant de préférence effectuée à une température entre 20 et 70°C, et les granules étant ensuite, au besoin, séchés.